(19) Europäisches Patentamt
European Patent Office
Office européen des brevets

(11) Numéro de publication: **0 046 719**
**A1**

# (12) DEMANDE DE BREVET EUROPEEN

(21) Numéro de dépôt: 81420124.0

(22) Date de dépôt: 21.08.81

(51) Int. Cl.$^3$: C 07 C 41/09
C 07 C 43/205, C 07 C 43/225

(30) Priorité: 25.08.80 FR 8018723

(43) Date de publication de la demande: 03.03.82 Bulletin 82/9

(84) Etats contractants désignés: BE CH DE GB IT LI NL

(71) Demandeur: RHONE-POULENC RECHERCHES
Brevets Pharma 25 Quai Paul Doumer
F-92408 Courbevoie Cedex(FR)

(72) Inventeur: Ratton, Serge
Vaulx Milieu
F-38290 - La Verpilliere(FR)

(74) Mandataire: Rioufrays, Roger et al,
RHONE-POULENC INDUSTRIES Centre de Recherches
des Carrières Service Brevets
F-69190 Saint-Fons(FR)

## (54) Procédé d'éthérification de phénols.

(57) L'invention concerne un nouveau procédé d'éthérification d'au moins une fonction phénolique de mono ou de polyphénols.

Le procédé consiste essentiellement dans la réaction d'un composé phénolique avec un agent d'éthérification choisi dans le groupe constitué par les alcools aliphatiques saturés ayant 1 à 6 atomes de carbone ou insaturés ayant 3 à 6 atomes de carbone, ledit procédé se caractérisant en ce que l'on opère en présence d'un sel d'acide carboxylique.

Les éthers arylaliphatiques obtenus par le procédé selon l'invention peuvent soit être utilisés directement, par exemple dans l'industrie pharmaceutique, soit servir d'intermédiaires pour la synthèse de composés plus complexes.

EP 0 046 719 A1

# PROCEDE D'ETHERIFICATION DE PHENOLS

La présente invention concerne un nouveau procédé d'éthérification d'au moins une fonction phénolique de mono- ou de polyphénols.

Il est connu d'éthérifier les fonctions phénoliques à l'aide d'un sulfate d'alkyle ou d'un halogénure d'alkyle (HOUBEN-WEYL Methoden der Organischen Chemie, Vol. III, page 54 (1965)).

Cette méthode donne de bons résultats mais les réactifs mis en oeuvre sont chers et, en outre, la réaction donne naissance à des sels minéraux posant des problèmes de corrosion des appareillages et de toxicité des effluents.

La demande de brevet français n° 74-18.172 (publiée sous le numéro 2.231.649) décrit un procédé d'éthérification de composés phénoliques, ayant un ou plusieurs groupements hydroxyle, par un alcool aliphatique saturé ayant 1 à 4 atomes de carbone ou un ester d'un tel alcool et d'un acide aliphatique saturé carboxylique, en présence d'une amine tertiaire aliphatique ou d'un chlorure, d'un sulfate ou d'un carboxylate d'une telle amine. Cependant ce procédé nécessite des opérations délicates de séparation des réactifs et catalyseurs en fin de réaction.

Enfin la demande de brevet français n° 77-37263 (publiée sous le n° 2 373 506) décrit un procédé de préparation d'oxydes d'alkyle et d'aryle par réaction, en présence de résines échangeuses de cations fortement acides, d'un composé aromatique hydroxylé avec un alcool aliphatique, ce procédé se caractérisant en ce que l'on opère avec au moins 3 moles de composé aromatique hydroxylé par mole d'alcool aliphatique. La température de mise en oeuvre du procédé est située de préférence entre 110°C et 130°C. Le principal inconvénient d'un tel procédé réside dans la cherté des résines utilisées comme catalyseur et dans leur sensibilité à la chaleur qui risque de provoquer leur dégradation.

Il a maintenant été trouvé un procédé nouveau et simple, constituant la présente invention, qui met en jeu des réactifs peu coûteux et qui permet l'éthérification avec un bon rendement d'au moins

une fonction phénolique de mono- ou de polyphénols.

Plus précisément, l'invention a pour objet un nouveau procédé d'éthérification d'au moins une fonction phénolique d'un composé de formule générale :

$$HO - Ar - (R)_n \qquad (I)$$

dans laquelle :

- Ar représente un radical aromatique constitué par un cycle benzénique ou par une structure formée par plusieurs cycles benzéniques orthocondensés ou ortho- et péricondensés,

- les substituants R, identiques ou différents, représentent un groupement hydroxyle, un radical alkyle linéaire ou ramifié ayant de 1 à 6 atomes de carbone, un radical alkényle linéaire ou ramifié ayant 2 à 6 atomes de carbone, un radical phényle éventuellement substitué, un radical cycloalkyle éventuellement substitué, un radical phénylalkyle dans lequel la chaîne aliphatique comporte 1 à 4 atomes de carbone, un radical cycloalkylalkyle dans lequel la chaîne aliphatique comporte de 1 à 4 atomes de carbone, un radical alkoxy ayant de 1 à 4 atomes de carbone, un atome d'halogène, un groupement nitro, un groupement amine, un groupement aldéhyde -CHO, un groupement nitrile, un groupement hydroxycarbonyle, un groupement alkoxycarbonyle de formule $-COOR_1$ dans laquelle $R_1$ est un radical alkyle ayant 1 à 6 atomes de carbone, alkényle ayant 2 à 6 atomes de carbone, cycloalkyle ayant 5 ou 6 atomes de carbone et éventuellement substitué, phényle éventuellement substitué ou un enchaînement de plusieurs de ces radicaux ou un groupement acyle de formule $-CO-R_2$ dans laquelle $R_2$ est un radical alkyle ayant 1 à 6 atomes de carbone, cycloalkyle ayant 5 ou 6 atomes de carbone et éventuellement substitué, phényle éventuellement substitué ou un enchaînement de plusieurs de ces radicaux.

- n est un nombre de 0 à 5,

par réaction avec un agent d'éthérification choisi dans le groupe constitué par les alcools aliphatiques, linéaires ou ramifiés, soit saturés ayant de 1 à 6 atomes de carbone, soit insaturés ayant de 3 à 6 atomes de carbone, caractérisé en ce que l'on opère en présence d'un sel

d'acide carboxylique.

L'invention peut également être présentée comme un procédé de préparation d'éthers de phénol par réaction d'un composé de formule générale (I) avec un agent d'éthérification choisi dans le groupe constitué par les alcools aliphatiques linéaires ou ramifiés, soit saturés ayant de 1 à 6 atomes de carbone, soit insaturés ayant de 3 à 6 atomes de carbone, ledit procédé se caractérisant en ce que l'on opère en présence d'un sel d'acide carboxylique.

De manière préférée, on applique le procédé à des composés phénoliques de formule (I) dans laquelle :

- le radical Ar correspond au benzène, au naphtalène, à l'anthracène ou au phénanthrène,

- les substituants R, identiques ou différents, représentent un groupement hydroxyle ; un radical alkyle linéaire ou ramifié ayant 1 à 4 atomes de carbone tel que méthyle, éthyle, isopropyle, n-propyle, n-butyle, isobutyle ou tertiobutyle ; un radical alkényle linéaire ou ramifié ayant 2 à 4 atomes de carbone, tel que vinyle, allyle, propène-1 yle, isopropényle, butène-1 yle, butène-2 yle, butène-3 yle, méthyl-1 propène-1 yle, méthyl-1 propène-2 yle, méthyl-2 propène-1 yle, méthyl-2 propène-2 yle ; un radical phényle éventuellement substitué ; un radical cyclohexyle éventuellement substitué ; un radical phénylalkyle dans lequel la chaîne aliphatique comporte de 1 à 3 atomes de carbone tel que benzyle, phénéthyle, phénylpropyle et phénylisopropyle ; un radical cyclohexylalkyle dans lequel la chaîne aliphatique comporte 1 à 3 atomes de carbone ; un radical alkoxy ayant 1 ou 2 atomes de carbone tel que méthoxy et éthoxy ; un atome de chlore, un atome de brome ; un groupement nitro ; un groupement aldéhyde ; un groupement hydroxycarbonyle ; un groupement nitrile ; un groupement alkoxycarbonyle de formule $-COOR_1$ dans laquelle $R_1$ est un radical alkyle ayant 1 à 4 atomes de carbone tel que ceux indiqués ci-avant, un radical alkényle ayant 2 à 4 atomes de carbone tel que ceux indiqués ci-avant, un radical cyclohexyle éventuellement substitué, un radical phényle éventuellement substitué ou un enchaînement de plusieurs de ces radicaux ; un groupement acyle de formule $-CO-R_2$ dans laquelle $R_2$ est un radical alkyle ayant 1 à 4 atomes de carbone, un radical cyclohexyle éventuellement substitué, un

radical phényle éventuellement substitué tels que ceux indiqués ci-avant ou un enchaînement de plusieurs de ces radicaux ;

- n est un nombre entier de 0 à 3.

A titre d'exemples de tels composés phénoliques, on peut citer :

- les monophénols comme le phénol, le naphtol-1, le naphtol-2, le phénanthrol-1, le phénanthrol-2, le phénanthrol-3, le phénanthrol-9, l'anthrol-1, l'anthrol-2, l'anthrol-9
- les diphénols comme le résorcinol, la pyrocatéchol, l'hydroquinone, le dihydroxy-1,2 naphtalène, le dihydroxy-1,3 naphtalène, le dihydroxy-1,4 naphtalène, le dihydroxy-1,5 naphtalène, le dihydroxy-1,6 naphtalène, le dihydroxy-1,7 naphtalène, le dihydroxy-1,8 naphtalène, le dihydroxy-2,3 naphtalène, le dihydroxy-2,6 naphtalène, le dihydroxy-2,7 naphtalène, le dihydroxy-1,2 anthracène, le dihydroxy-1,5 anthracène, le dihydroxy-1,8 anthracène, le dihydroxy-2,6 anthracène, le dihydroxy-9,10 anthracène, le dihydroxy-3,4 phénanthrène,
- les triphénols comme le pyrogallol, le trihydroxy-1,2,4 benzène, le trihydroxy-1,3,5 benzène, le trihydroxy-1,2,9 anthracène, le trihydroxy-1,2,10 anthracène, le trihydroxy-1,4,9 anthracène, le trihydroxy-1,5,9 anthracène, le trihydroxy-1,8,9 anthracène, le trihydroxy-2,3,9 anthracène, le trihydroxy-3,4,5 phénanthrène,
- les mono- ou diphénols portant également 1 ou plusieurs autres substituants R comme : le chloro-2 phénol, le chloro-3 phénol, le chloro-4 phénol, le bromo-2 phénol, le bromo-3 phénol, le bromo-4 phénol, le nitro-2 phénol, le nitro-3 phénol, le nitro-4 phénol, le méthyl-2 phénol, le méthyl-3 phénol, le méthyl-4 phénol, l'éthyl-2 phénol, l'éthyl-3 phénol, l'éthyl-4 phénol, l'isopropyl-2 phénol, l'isopropyl-3 phénol, l'isopropyl-4 phénol, le propyl-2 phénol, le propyl-3 phénol, le propyl-4 phénol, le (propène-1 yle)-4 phénol, l'allyl-2 phénol, l'allyl-4 phénol, le butyl-3 phénol, le butyl-4 phénol, l'isobutyl-4 phénol, le tertiobutyl-2 phénol, le tertiobutyl-3 phénol, le tertiobutyl-4 phénol, le benzyl-2 phénol, le benzyl-4 phénol, le cyclohexyl-2 phénol, le méthoxy-2 phénol, le méthoxy-3 phénol, le méthoxy-4 phénol, l'éthoxy-2 phénol, l'éthoxy-3 phénol, l'éthoxy-4 phénol, le dichloro-2,3 phénol, le dichloro-2,4 phénol, le dichloro-2,5 phénol, le dichloro-2,6 phénol, le dichloro-3,4 phénol, le

dichloro-3,5 phénol, le diméthyl-1,2 hydroxy-3 benzène, le diméthyl-1,2 hydroxy-4 benzène, le diméthyl-1,3 hydroxy-5 benzène, le diméthyl-1,3 hydroxy-2 benzène, le diméthyl-1,4 hydroxy-2 benzène, le diméthyl-2,4 hydroxy-1 benzène, le tertiobutyl-1 hydroxy-2 méthyl-4 benzène, le tertiobutyl-2 hydroxy-1 méthyl-4 benzène, le tertiobutyl-2 éthyl-4 hydroxy-1 benzène, le tertiobutyl-4 éthyl-2 hydroxy-1 benzène, le ditertiobutyl-1,3 hydroxy-2 benzène, le ditertiobutyl-2,4 hydroxy-1 benzène, l'hydroxy-2 isopropyl-4 méthyl-1 benzène, l'allyl-2 chloro-4 hydroxy-1 benzène, l'hydroxy-1 triméthyl-2,4,5 benzène, l'hydroxy-2 triméthyl-1,3,5 benzène, l'hydroxy-2 tritertiobutyl-1,3,5 benzène, le ditertiobutyl-1,3 hydroxy-2 méthyl-5 benzène, le ditertiobutyl-1,5 hydroxy-2 méthyl-3 benzène, le ditertiobutyl-1,5 hydroxy-2 méthyl-4 benzène, le tertiobutyl-1 diméthyl-2,5 hydroxy-4 benzène, le tertiobutyl-1 diméthyl-3,5 hydroxy-2 benzène, le tertiobutyl-1 diméthyl-4,5 hydroxy-2 benzène, le tertiobutyl-5 diméthyl-1,3 hydroxy-2 benzène, le chloro-1 diméthyl-2,3 hydroxy-4 benzène, le chloro-1 diméthyl-2,3 hydroxy-5 benzène, le chloro-1 diméthyl-2,4 hydroxy-5 benzène, le chloro-1 diméthyl-2,5 hydroxy-4 benzène, le chloro-1 diméthyl-3,4 hydroxy-2 benzène, le chloro-1 diméthyl-4,5 hydroxy-2 benzène, le chloro-2 diméthyl-1,3 hydroxy-5 benzène, le chloro-2 diméthyl-1,5 hydroxy-3 benzène, le chloro-2 diméthyl-3,4 hydroxy-1 benzène, le chloro-5 diméthyl-1,3 hydroxy-2 benzène, le diméthyl-1,2 hydroxy-3 nitro-5 benzène, le diméthyl-1,2 hydroxy-4 nitro-5 benzène, le diméthyl-1,3 hydroxy-2 nitro-4 benzène, le diméthyl-1,3 hydroxy-2 nitro-5 benzène, le diméthyl-1,4 hydroxy-2 nitro-3 benzène, le diméthyl-1,4 hydroxy-2 nitro-5 benzène, le diméthyl-1,5 hydroxy-2 nitro-3 benzène, le diméthyl-1,5 hydroxy-3 nitro-2 benzène, le diméthyl-2,5 hydroxy-1 nitro-3 benzène, le nitro-8 naphtol-1, le nitro-1 naphtol-2, le nitro-5 naphtol-2, le méthyl-1 naphtol-2, le bromo-1 dihydroxy-2,4 benzène, le bromo-1 dihydroxy-3,5 benzène, le bromo-2 dihydroxy-1,3 benzène, le bromo-2 dihydroxy-1,4 benzène, le bromo-4 dihydroxy-1,2 benzène, le butyl-1 dihydroxy-2,4 benzène, le chloro-1 dihydroxy-2,3 benzène, le chloro-1 dihydroxy-2,4 benzène, le chloro-1 dihydroxy-3,5 benzène, le chloro-2 dihydroxy-1,3 benzène, le chloro-2 dihydroxy-1,4 benzène, le chloro-4 dihydroxy-1,2 benzène, le

dihydroxy-2,4 éthyl-1 benzène, le dihydroxy-2,4 isobutyl-1 benzène, le dihydroxy-1,2 isopropyl-4 benzène, le dihydroxy-1,4 isopropyl-2 benzène, le dihydroxy-2,4 isopropyl-1 benzène, le dihydroxy-2,3 isopropyl-1 méthyl-4 benzène, le dihydroxy-1,4 isopropyl-2 méthyl-5 benzène, le dihydroxy-1,2 méthyl-3 benzène, le dihydroxy-1,3 méthyl-2 benzène, le dihydroxy-1,3 nitro-2 benzène, le dihydroxy-1,4 nitro-2 benzène, le dihydroxy-1,2 propyl-4 benzène, le dihydroxy-1,3 propyl-5 benzène, le dihydroxy-2,4 propyl-1 benzène, le dihydroxy-3,4 benzaldéhyde, l'hydroxy-3 méthoxy-4 benzaldéhyde, l'hydroxy-4 méthoxy-3 benzaldéhyde (ou vanilline), le dichloro-1,2 dihydroxy-4,5 benzène, le dichloro-1,3 dihydroxy-2,5 benzène, le dichloro-1,4 dihydroxy-2,5 benzène, le dichloro-1,5 dihydroxy-2,3 benzène, le dichloro-1,5 dihydroxy-2,4 benzène, le dichloro-2,3 dihydroxy-1,4 benzène, le dihydroxy-1,2 diméthyl-3,5 benzène, le dihydroxy-1,2 diméthyl-4,5 benzène, le dihydroxy-1,3 diméthyl-2,4 benzène, le dihydroxy-1,3 diméthyl-2,5 benzène, le dihydroxy-1,4 diméthyl-2,3 benzène, le dihydroxy-1,4 diméthyl-2,5 benzène, le dihydroxy-1,5 diméthyl-2,4 benzène, le dihydroxy-1,5 diméthyl-3,4 benzène, le dihydroxy-2,5 diméthyl-1,3 benzène, le dihydroxy-1,3 dinitro-2,4 benzène, le dihydroxy-4,5 nitro-2 benzaldéhyde, l'hydroxy-4 méthoxy-5 nitro-2 benzaldéhyde, l'hydroxy-5 méthoxy-4 nitro-2 benzaldéhyde.

Le procédé selon l'invention est plus particulièrement appliqué au phénol, au naphtol-1, au naphtol-2, au méthyl-2 phénol, au méthyl-3 phénol, au méthyl-4 phénol, aux monochlorophénols, aux dichlorophénols, aux monoéthylphénols, au pyrocatéchol, au résorcinol, à l'hydroquinone, au méthoxy-2 phénol, au méthoxy-3 phénol, au méthoxy-4 phénol, à l'éthoxy-2 phénol, à l'éthoxy-3 phénol, à l'éthoxy-4 phénol, au dihydroxy-3,4 benzaldéhyde, à l'hydroxy-4 méthoxy-3 benzaldéhyde, au dihydroxy-4,5 nitro-2 benzaldéhyde, à l'hydroxy-4 méthoxy-5 nitro-2 benzaldéhyde, à l'hydroxy-5 méthoxy-4 nitro-2 benzaldéhyde, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

La concentration du composé phénolique de formule (I) dans le

milieu réactionnel n'est pas critique. Elle varie très largement, notamment en fonction de la solubilité de ce composé dans ledit milieu, qui est constitué par l'agent d'éthérification, le sel d'acide carboxylique et le cas échéant un tiers solvant ou un diluant.

Pour des raisons de commodité, la concentration du composé phénolique sera exprimée par rapport au milieu liquide, c'est à dire au milieu réactionnel dont on exclut le composé phénolique lui-même et le sel d'acide carboxylique.

C'est ainsi que généralement on opère avec de 1 % à 50 % en poids de composé phénolique par rapport au volume du milieu liquide. Le plus fréquemment cette concentration est comprise entre 2 % et 30 % en poids par volume.

Parmi les agents d'éthérification que l'on peut mettre en oeuvre dans le procédé selon l'invention, on peut citer les alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

La quantité d'agent d'éthérification utilisée est généralement choisie de telle façon que le rapport molaire alcool sur composé phénolique de formule (I) est égal ou supérieur à 0,5 et de préférence égal ou supérieur à 1.

Les agents d'éthérification que l'on préfère utiliser généralement sont le méthanol, l'éthanol, le n-propanol, le propène-2 ol-1 et le méthyl-2 propène-2 ol-1.

Enfin parmi ces agents d'éthérification préférés, on préfère plus particulièrement employer le méthanol et l'éthanol.

L'agent d'éthérification peut constituer le milieu solvant dans lequel s'opère la réaction d'éthérification selon le procédé de l'invention. C'est la solution adoptée le plus fréquemment. Mais il est bien entendu que l'on peut également utiliser un tiers solvant ou un diluant, liquide dans les conditions de mise en oeuvre du procédé, dans la mesure où ledit tiers solvant ou ledit diluant est inerte vis à vis des réactifs et stable aux températures auxquelles est effectuée la réaction.

A titre d'exemples de tels tiers solvants ou diluants, on peut

citer les hydrocarbures aliphatiques, cyclaniques ou aromatiques et l'eau.

Le sel d'acide carboxylique servant de catalyseur dans le procédé selon l'invention peut être tout carboxylate, notamment les carboxylates des métaux alcalins, d'ammonium et des métaux alcalino-terreux, de préférence les carboxylates de métaux alcalins. A titre d'exemples, on peut citer notamment les carboxylates de sodium, de potassium, de lithium et d'ammonium ; on peut également citer les carboxylates de calcium, de magnésium et de baryum.

Les acides carboxyliques servant à obtenir de tels carboxylates peuvent être les acides mono- ou polyfonctionnels, aliphatiques saturés ou insaturés, aromatiques, arylaliphatiques, cycloaliphatiques dont les cycles peuvent être substitués par un ou plusieurs radicaux.

On peut citer à titre d'illustration des acides carboxyliques précédents des monoacides aliphatiques saturés comme l'acide acétique, le propanoïque, le n-butanoïque, le méthyl-2 propanoïque, le n-pentanoïque, le méthyl-2 butanoïque, le méthyl-3 butanoïque, le diméthyl-3,3 butanoïque, le n-hexanoïque, le méthyl-2 pentanoïque, le méthyl-3 pentanoïque, le méthyl-4 pentanoïque ; des monoacides aliphatiques insaturés comme le propènoïque, le butène-2 oïque, le méthyl-2 propènoïque, le butène-3 oïque, le méthyl-2 butène-2 oïque cis (acide angélique), le méthyl-2 butène-2 oïque trans (acide tiglique), le pentène-4 oïque, l'hexène-3 oïque, l'hexène-4 oïque ; des diacides aliphatiques saturés comme l'acide malonique, l'acide succinique, l'acide glutarique, l'acide adipique ; des diacides éthyléniques comme l'acide maléïque et l'acide fumarique ; des mono- ou des diacides aromatiques comme l'acide benzoïque, l'acide orthophtalique, l'acide isophtalique, l'acide téréphtalique, les acides mononitrobenzoïques, les acides monochlorobenzoïques ; des acides arylaliphatiques comme l'acide phénylacétique, le phényl-2 propanoïque, le phényl-4 propanoïque ; des acides cycloaliphatiques comme l'acide cyclohexanedicarboxylique-1,3, l'acide cyclohexanedicarboxylique-1,4.

Parmi tous les carboxylates que l'on peut utiliser on préfère généralement les sels des acides carboxyliques aliphatiques saturés, monofonctionnels ayant de 2 à 6 atomes de carbone, tels que notamment l'acide acétique, le propanoïque, le n-butanoïque, le n-pentanoïque, le

n-hexanoïque, le méthyl-2 propanoïque, le méthyl-2 butanoïque, le méthyl-3 butanoïque, le diméthyl-3,3 butanoïque, le méthyl-2 pentanoïque, le méthyl-3 pentanoïque et le méthyl-4 pentanoïque, ou difonctionnels ayant 3 à 6 atomes de carbone, tels que notamment l'acide malonique, l'acide succinique, l'acide glutarique et l'acide adipique, les sels de l'acide benzoïque et ceux des acides orthophtalique, isophtalique et téréphtalique. Parmi les sels de ces acides on préfère les sels de métaux alcalins.

De manière encore préférée, on emploie les sels de sodium ou de potassium de ces acides. Parmi ces derniers sels, on utilise préférentiellement l'acétate de sodium, le propionate de sodium et le succinate de sodium.

La quantité de sel d'acide carboxylique présent dans le milieu peut varier dans de larges limites. Si on exprime cette quantité par rapport au composé phénolique, elle n'est généralement pas inférieure à 0,1 fois le poids dudit composé phénolique. La quantité maximale n'est pas critique. Habituellement elle ne dépasse pas 50 fois le poids du composé phénolique. On préfère le plus souvent utiliser des rapports pondéraux sel d'acide carboxylique/composé phénolique variant de 0,5 à 20.

Sur un plan pratique, afin d'obtenir un très bon rendement en éther par rapport au composé phénolique transformé, tout en ayant une mise en oeuvre et un traitement final du mélange réactionnel faciles, on choisit de préférence la réalisation suivante du procédé selon l'invention. On opère généralement dans un milieu constitué par l'alcool qui joue le double rôle de réactif et de solvant ; on ajoute également le plus souvent l'acétate de sodium.

Les quantités relatives des divers réactifs ou constituants du mélange réactionnel sont alors choisies dans les zones préférentielles indiquées précédemment.

Le procédé selon l'invention nécessite pour sa mise en oeuvre un chauffage des réactifs ; la température à laquelle on opère peut varier de 150°C à 350°C. Elle se situe de préférence entre 220°C et 300°C.

La pression n'est pas un paramètre critique de la réaction. Habituellement elle est constituée par la pression autogène obtenue par chauffage, à la température désirée, du mélange réactionnel dans un

appareillage adéquat fermé. Elle se situe généralement entre 10 bars et 100 bars. Mais elle peut atteindre des valeurs plus élevées car il est possible, sans sortir du cadre de l'invention, de créer dans l'appareil utilisé pour la réaction, une pression initiale à froid supérieure à la pression atmosphérique, par exemple au moyen d'un gaz inerte tel que l'azote.

L'appareillage utilisé n'est pas spécifique au procédé de l'invention. Simplement il doit présenter certaines caractéristiques : il doit pouvoir résister aux pressions que l'on atteint lors du chauffage, il doit être étanche et évidemment ne pas être attaqué par les réactifs utilisés.

Pratiquement le procédé selon l'invention peut être mis en oeuvre de la manière suivante : on charge les différents constituants du mélange réactionnel, tels que définis précédemment, dans l'appareillage adéquat. On chauffe à la température désirée, de préférence sous agitation mais sans que celà soit véritablement indispensable, pendant une durée pouvant varier de quelques minutes à plus de 20 heures par exemple. Cependant cette durée est généralement de l'ordre de quelques heures, par exemple de 2 heures à 10 heures selon la température à laquelle on opère.

En fin de réaction, l'appareil est refroidi et la masse réactionnelle finale est traitée de manière classique, selon les réactifs utilisés ; si le milieu contient de l'eau, les composés organiques autres que le sel d'acide carboxylique sont extraits par un solvant non miscible à l'eau. Si le milieu ne contient pas ou très peu d'eau, on peut généralement séparer le sel d'acide carboxylique par filtration, soit directement, soit après l'avoir précipité par addition d'un solvant organique dans lequel il n'est pas soluble, mais qui dissout les composés formés lors de la réaction. On peut aussi rajouter de l'eau au milieu avant de procéder à l'extraction des composés organiques.

Les produits obtenus sont séparés, notamment du composé phénolique n'ayant pas été transformé, par des opérations courantes dans le domaine de la chimie, puis dosés si nécessaire, également par des méthodes bien connues de l'homme du métier.

Les éthers obtenus par le procédé selon l'invention peuvent soit être utilisés directement, soit servir d'intermédiaires pour la synthèse

de composés plus complexes, notamment pour la parfumerie, la pharmacie ou l'agriculture.

L'anisole par exemple peut être utilisé directement en parfumerie, ou servir d'intermédiaire de synthèse.

Dans les exemples qui vont suivre les dosages, sauf mention contraire, sont effectués par chromatographie gaz/liquide.

EXEMPLE 1

Dans un tube de verre résistant à la pression, on introduit les réactifs suivants :

- Acétate de sodium anhydre : 2,3 g
- Méthanol : 10 ml
- Phénol : 0,5086 g.

Le tube est scellé, puis on chauffe à 230°C sous agitation et on maintient à cette température pendant 4 heures.

En fin d'essai, on refroidit et on filtre l'acétate de sodium. On dose les produits par chromatographie gaz/liquide. On trouve :

- Phénol non transformé : 0,2385 g, soit un taux de transformation (TT) du phénol de 53,1 %.
- Anisole formée : 0,2657 g, soit un rendement par rapport au phénol transformé (RT) de 85,6 %.

EXEMPLE 2

Dans un tube de verre résistant à la pression, on introduit les réactifs suivants :

- Acétate de sodium anhydre : 2,3 g
- Méthanol : 10 ml
- Parachlorophénol : 0,5357 g.

Le tube est scellé, puis chauffé sous agitation à 230°C et maintenu 4 heures à cette température.

Le mélange réactionnel final est traité et dosé comme dans l'exemple 1 :

- parachlorophénol non transformé : 0,1444 g - TT = 73,05 %
- parachloroanisole formée : 0,3423 g - RT = 78,9 %.

EXEMPLE 3

Dans un tube de verre résistant à la pression, on introduit les réactifs suivants :

- Acétate de sodium anhydre : 4 g
- Méthanol : 10 ml
- Pyrocatéchol : 0,5 g.

Le tube est scellé, puis on chauffe à 250°C sous agitation et on maintient à cette température pendant 3 heures.

Le traitement du mélange réactionnel final et le dosage sont les mêmes que dans l'exemple 1.

On trouve les résultats suivants :

- TT du pyrocatéchol : 66 %
- RT en gaïacol : 42,7 %
- RT en vératrol : 45,4 %.

REVENDICATIONS

1°) Procédé d'éthérification d'au moins une fonction phénolique d'un composé de formule générale (I) :

$$HO - Ar - (R)_n \qquad (I)$$

dans laquelle :

- Ar représente un radical aromatique constitué par un cycle benzénique ou par plusieurs cycles benzéniques orthocondensés ou ortho- et péricondensés,
- les substituants R, identiques ou différents, représentent un groupement hydroxyle, un radical alkyle linéaire ou ramifié ayant de 1 à 6 atomes de carbone, un radical alkényle linéaire ou ramifié ayant 2 à 6 atomes de carbone, un radical phényle éventuellement substitué, un radical cycloalkyle éventuellement substitué, un radical phénylalkyle dans lequel la chaîne aliphatique comporte 1 à 4 atomes de carbone, un radical cycloalkylalkyle dans lequel la chaîne aliphatique comporte de 1 à 4 atomes de carbone, un radical alkoxy ayant de 1 à 4 atomes de carbone, un atome d'halogène, un groupement nitro, un groupement amine, un groupement aldéhyde, un groupement nitrile, un groupement hydroxycarbonyle, un groupement alkoxycarbonyle de formule $-COOR_1$ (dans laquelle $R_1$ est un radical alkyle ayant 1 à 6 atomes de carbone, alkényle ayant 2 à 6 atomes de carbone, cycloalkyle ayant 5 ou 6 atomes de carbone et éventuellement substitué, phényle éventuellement substitué ou un enchaînement de plusieurs de ces radicaux) ou un groupement acyle de formule $-CO-R_2$ (dans laquelle $R_2$ est un radical alkyle ayant 1 à 6 atomes de carbone, cycloalkyle ayant 5 ou 6 atomes carbone et éventuellement substitué, phényle éventuellement substitué ou un enchaînement de plusieurs de ces radicaux),
- n est un nombre de 0 à 5,

par réaction avec un agent d'éthérification choisi dans le groupe constitué par les alcools aliphatiques linéaires ou ramifiés, soit saturés ayant de 1 à 6 atomes de carbone, soit insaturés ayant de 3 à 6

atomes de carbone,
caractérisé en ce que l'on opère en présence d'un sel d'acide carboxylique.

2°) Procédé selon la revendication 1, caractérisé en ce qu'il est appliqué à un composé de formule (I) dans laquelle :

- le radical Ar correspond au benzène, au naphtalène, à l'anthracène ou au phénanthrène
- les substituants R, identiques ou différents, représentent un groupement hydroxyle ; un radical alkyle linéaire ou ramifié ayant de 1 à 4 atomes de carbone ; un radical alkényle linéaire ou ramifié ayant 2 à 4 atomes de carbone ; un radical phényle éventuellement substitué ; un radical cyclohexyle éventuellement substitué ; un radical phénylalkyle dans lequel la chaîne aliphatique comporte 1 à 3 atomes de carbone ; un radical cyclohexylalkyle dans lequel la chaîne aliphatique comporte 1 à 3 atomes de carbone ; un radical alkoxy ayant 1 ou 2 atomes de carbone ; un atome de chlore ; un atome de brome ; un groupement nitro ; un groupement aldéhyde -CHO ; un groupement hydroxycarbonyle ; un groupement nitrile ; un groupement alkoxycarbonyle de formule $-COOR_1$ dans laquelle $R_1$ est un radical alkyle ayant 1 à 4 atomes de carbone, un radical alkényle ayant 2 à 4 atomes de carbone, un radical cyclohexyle éventuellement substitué, un radical phényle éventuellement substitué ou un enchaînement de plusieurs de ces radicaux ; un groupement acyle de formule $-CO-R_2$ dans laquelle $R_2$ est un radical alkyle ayant 1 à 4 atomes de carbone, un radical cyclohexyle éventuellement substitué, un radical phényle éventuellement substitué ou un enchaînement de plusieurs de ces radicaux,
- n est un nombre entier de 0 à 3.

3°) Procédé selon l'une des revendications 1 et 2, caractérisé en ce qu'il est appliqué au phénol, au naphtol-1, au naphtol-2, au méthyl-2 phénol, au méthyl-3 phénol, au méthyl-4 phénol, aux monochlorophénols, aux dichlorophénols, au monoéthylphénols, au mononitrophénols, au pyrocatéchol, au résorcinol, à l'h droquinone, au méthoxy-2 phénol, au méthoxy-3 phénol, au méthoxy-4 phénol, à l'éthoxy-2 phénol, à l'éthoxy-3 phénol, à l'éthoxy-4 phénol, au dihydroxy-3,4 benzaldéhyde, à l'hydroxy-4

méthoxy-3 benzaldéhyde, au dihydroxy-4,5 nitro-2 benzaldéhyde, à l'hydroxy-4 méthoxy-5 nitro-2 benzaldéhyde, à l'hydroxy-5 méthoxy-4 nitro-2 benzaldéhyde, au dihydroxy-1,2 naphtalène, au dihydroxy-1,3 naphtalène, au dihydroxy-1,4 naphtalène, au dihydroxy-1,5 naphtalène, au dihydroxy-1,6 naphtalène, au dihydroxy-1,7 naphtalène, au dihydroxy-1,8 naphtalène, au dihydroxy-2,3 naphtalène, au dihydroxy-2,6 naphtalène, au dihydroxy-2,7 naphtalène.

4°) Procédé selon l'une quelconque des revendications 1 à 3, caractérisé en ce que l'agent d'éthérification est choisi parmi les alcools tels que le méthanol, l'éthanol, le n-propanol, l'isopropanol, le butanol-1, le butanol-2, le tertiobutanol, le propène-2 ol-1, le méthyl-2 propène-2 ol-1, le butène-2 ol-1, le butène-3 ol-1, le butène-3 ol-2.

5°) Procédé selon l'une quelconque des revendications 1 à 4, caractérisé en ce que le sel d'acide carboxylique utilisé est un carboxylate de métal alcalin, d'ammonium ou de métal alcalino-terreux, et de préférence de métal alcalin.

6°) Procédé selon l'une quelconque des revendications 1 à 5, caractérisé en ce que le sel d'acide carboxylique utilisé est un sel d'un acide aliphatique saturé monofonctionnel ayant 2 à 6 atomes de carbone ou difonctionnel ayant 3 à 6 atomes de carbone, de l'acide benzoïque ou de l'un des acides ortho-, méta- et téréphtaliques.

7°) Procédé selon l'une quelconque des revendications 1 à 6, caractérisé en ce que l'on opère à une température située entre 150°C et 350°C, et de préférence entre 220°C et 300°C.

8°) Procédé selon l'une quelconque des revendications 1 à 7, caractérisé en ce que l'on utilise un rapport molaire alcool/composé phénolique égal ou supérieur à 0,5, et de préférence égal ou supérieur à 1.

0046719

Numéro de la demande

Office européen des brevets

# RAPPORT DE RECHERCHE EUROPEENNE

EP 81 42 0124

| DOCUMENTS CONSIDERES COMME PERTINENTS | | | CLASSEMENT DE LA DEMANDE (Int. Cl.³) |
|---|---|---|---|
| Catégorie | Citation du document avec indication, en cas de besoin, des parties pertinentes | Revendication concernée | |
| A | CHEMICAL ABSTRACTS, vol. 78, 1973 page 444, résumé no. 42937b Columbus, Ohio, US T. KITO et al.: "O-Alkylation of phenols by esters"<br>& Bull. Chem. Soc. Jap. 1972, 45(11), 3490-2<br>* Résumé en entier *<br>-- | 1 | C 07 C 41/09<br>43/205<br>43/225 |
| A | CHEMICAL ABSTRACTS, vol. 88, no.17 24 avril 1978, page 505, résumé no. 120721z Columbus, Ohio, US N.S. KOZLOV et al.:"Alkylation of phenol by methanol and ethanol in the presence of polyphosphoric acid"<br>& Izv. Vyssh. Uchebn., Zaved., Khim. Khim. Tekhnol. 1978, 21(1), 23-6<br>* Résumé en entier *<br>-- | 1 | DOMAINES TECHNIQUES RECHERCHES (Int. Cl.³)<br>C 07 C 43/09<br>43/205<br>43/225 |
| DA | FR - A - 2 373 506 (HAARMANN & REIMER)<br>* Revendication 1 *<br>---- | 1 | CATEGORIE DES DOCUMENTS CITES<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons<br>&: membre de la même famille, document correspondant |
| X | Le présent rapport de recherche a été établi pour toutes les revendications | | |

| Lieu de la recherche | Date d'achèvement de la recherche | Examinateur |
|---|---|---|
| La Haye | 24-11-1981 | VERHULST |